# FASCICULE DE BREVET EUROPEEN

(11) **EP 3 316 935 B1**
(45) Date de publication et mention de la délivrance du brevet: **24.04.2019**
(21) Numéro de dépôt: 16744800.0
(22) Date de dépôt: 30.06.2016
(51) Int. Cl.: A61M 5/20, A61M 5/30

(54) **DISPOSITIF D'INJECTION SANS AIGUILLE ÉQUIPÉ D'UNE MEMBRANE A ÉTANCHÉITÉ AMÉLIORÉE**
NADELLOSE INJEKTIONSVORRICHTUNG MIT EINER VERBESSERTEN DICHTUNGSMEMBRAN
NEEDLELESS INJECTION DEVICE EQUIPPED WITH AN IMPROVED SEALING MEMBRANE

(30) Priorité: 30.06.2015 FR 1556163
(43) Date de publication de la demande: 09.05.2018
(73) Titulaire: Crossject, 21000 Dijon (FR)
(72) Inventeur: FRÉDÉRIC, Mabile, 21240 Talant (FR); STEINBERGER, Robin, Leobersdorf (AT)
(74) Mandataire: Chevalier, Renaud Philippe
(86) Numéro de dépôt international: PCT/FR2016/051657
(87) Numéro de publication internationale: WO 2017/001796

(56) Documents cités:
- EP-A1- 1 961 436
- WO-A1-97/21457
- FR-A1- 2 815 544
- FR-A1- 2 852 516

## Description

L'invention concerne un dispositif d'injection sans aiguille comportant une membrane souple à étanchéité améliorée.

Le domaine technique de l'invention est celui des dispositifs d'injection sans aiguille, pré-remplis et jetables, fonctionnant avec une source d'énergie comme par exemple un générateur de gaz, et utilisés pour les injections intradermiques, sous-cutanées et intramusculaires, de principe actif liquide à usage thérapeutique en médecine humaine ou vétérinaire.

Le principe actif est constitué par un liquide plus ou moins visqueux, un mélange de liquide, ou un gel. Le principe actif peut également être un solide mis en solution dans un solvant approprié pour l'injection ou être constitué d'un solide pulvérulent mis en suspension à une certaine concentration dans un liquide approprié. La granulométrie du principe actif doit alors être compatible avec le diamètre des conduits pour éviter de les obturer.

Un dispositif d'injection comporte de manière connue, comme par exemple dans la demande de brevet FR-A-2815544 (équivalente à WO 02/34317), un corps comprenant successivement un générateur de gaz, une chambre d'expansion, un réservoir contenant le principe actif liquide et un système d'injection.

Le réservoir est constitué par un tube en verre qui est inséré dans un logement tubulaire délimité par le corps du dispositif, et qui est obturé par un piston amont et un piston aval entre lesquels est contenu le principe actif liquide.

L'extrémité libre aval, ou inférieure, du réservoir, coopère avec une buse d'injection qui délimite au moins un canal d'injection s'étendant axialement suivant un axe d'injection.

La buse d'injection est délimitée axialement par une face supérieure en appui axial sur le réservoir, et une face inférieure d'injection adaptée pour coopérer avec un opercule de fermeture.

De plus, le dispositif d'injection comporte un capot creux qui enveloppe le corps et qui délimite une ouverture inférieure adaptée pour le passage de la buse d'injection.

Pour permettre l'injection du principe actif, le corps est monté coulissant dans le capot, de bas en haut suivant un axe de coulissement, entre une position de repos et une position d'injection, l'entraînement du corps étant réalisé lorsque l'utilisateur appui la buse d'injection sur sa peau.

Le déplacement du corps dans le capot permet le déclenchement du générateur de gaz, générant un gaz sous pression qui entraîne en déplacement les pistons pour injecter le principe actif à travers la peau du patient, en passant par la buse d'injection.

On connait un dispositif d'injection qui est équipé d'une membrane déformable élastiquement de forme globalement en T, qui comprend un disque annulaire radial qui est interposé axialement entre l'extrémité supérieure du réservoir et un siège formé par le corps, et une partie tubulaire qui s'étend axialement dans le réservoir, depuis le disque annulaire.

La partie tubulaire de la membrane est conçue pour s'étendre axialement sous l'effet du gaz sous pression, afin d'entraîner les pistons en déplacement.

Pour assurer l'étanchéité entre le réservoir et le corps du dispositif, notamment au moment de l'injection, le disque annulaire de la membrane est comprimé axialement entre l'extrémité supérieure du réservoir et le siège formé par le corps du dispositif.

A cet effet, la buse d'injection est vissée sur le corps, à l'extrémité libre du logement renfermant le réservoir, pour pousser axialement le réservoir contre le siège associé.

De façon surprenante, on constate que le disque annulaire de la membrane, une fois comprimé, peut fluer axialement et déborder entre le réservoir et la paroi tubulaire du logement.

Une telle déformation du disque risque de nuire à l'étanchéité entre la membrane et le corps du dispositif, provoaquant ainsi des fuites.

La présente invention vise notamment à résoudre cet inconvénient et se rapporte pour ce faire à un dispositif d'injection sans aiguille comportant :
- un corps formant un logement qui est délimité par une paroi tubulaire et qui s'étend axialement depuis un siège radial supérieur,
- un générateur de gaz,
- un réservoir tubulaire qui renferme un principe actif et qui s'étend axialement dans ledit logement depuis une extrémité supérieure formant une collerette supérieure, jusqu'à une extrémité inférieure,
- une membrane déformable élastiquement de forme globalement en T, qui comprend un disque annulaire radial qui est interposé axialement entre la collerette supérieure du réservoir et ledit siège formé par le corps, et une partie tubulaire qui s'étend axialement dans le réservoir, depuis le disque annulaire, et
- une buse d'injection du principe actif qui est agencée à l'extrémité inférieure du réservoir,
caractérisé en ce qu'il comporte une rondelle annulaire qui est interposée axialement entre le disque annulaire de la membrane et la collerette supérieure du réservoir, et qui s'étend radialement globalement jusqu'à la paroi tubulaire du logement du corps, pour limiter le fluage axiale du disque de la membrane entre le réservoir et la paroi tubulaire dudit logement, lorsque le disque de la membrane est comprimé axialement.

Ainsi, la rondelle permet de limiter l'espace à travers lequel la membrane peut s'épandre, ou fluer, espace qui est inhérent aux tolérances géométriques de fabrication du réservoir et du corps.

De préférence, la rondelle annulaire est délimitée radialement par un bord périphérique qui présente un jeu radial avec la paroi tubulaire du logement associé, le jeu radial étant compris entre 0,075 et 0,025 millimètre suivant le rayon de la rondelle.

Selon un exemple de réalisation, le disque de la membrane forme un cordon annulaire qui fait saillie axialement vers le haut et qui est logé dans une gorge de forme complémentaire qui est délimitée par le siège du corps.

Cette caractéristique permet d'améliorer l'étanchéité entre la membrane et le corps.

Avantageusement, pour favosriser l'étanchéité entre la membrane et le corps, le disque de la membrane est adapté pour être agencé sans jeu dans l'espace délimité entre le siège formé par le corps, la collerette supérieure du réservoir et la paroi tubulaire du logement formé par le corps.

Selon une autre caractéristique, le corps est réalisé par moulage en injection.

De plus, la membrane est réalisée en matériau déformable élastiquement à base d'élastomère.

Aussi, la buse d'injection est vissée sur une extrémité libre débouchante du logement formé par le corps, la buse comprimant axialement l'ensemble formé par le réservoir, la membrane et la rondelle sur le siège du logement.

Selon un exemple de réalisation préféré, la buse est vissée suivant un couple de serrage d'au moins 120 Newtons.

Aussi, le principe actif contenu dans le réservoir est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

D'autres caractéristiques et avantages de l'invention apparaîtront à la lecture de la description détaillée qui suit pour la compréhension de laquelle on se reportera aux dessins annexés dans lesquels :
- la figure 1 est une vue schématique d'ensemble en section transversale, qui illustre le dispositif d'injection selon l'invention ;
- la figure 2 est une vue de détail en section transversale de la figure 1, qui illustre la membrane et le rondelle dans un état comprimé axialement ;
- la figure 3 est une vue de détail en section transversale de la figure 1, qui illustre la membrane et la rondelle dans un état de repos avant compression ;
- la figure 4 est une vue de détail en section transversale qui illustre une membrane d'un dispositif d'injection sans aiguille selon l'art antérieur.

Dans la description et les revendications, pour clarifier la description et les revendications, on adoptera à titre non limitatif la terminologie longitudinal, vertical et transversal en référence au trièdre L, V, T indiqué aux figures.

De plus, dans la présente demande, les termes « supérieur », « inférieur », « horizontal », « vertical », et leurs dérivés font référence à la position ou à l'orientation d'un élément ou d'un composant, cette position ou cette orientation étant considérée en référence à l'orientation du dispositif sur les figures et au trièdre L, V, T, sans référence à la gravité terrestre.

De même, les termes « axial » et « radial » doivent s'entendre en référence à l'axe B d'injection du dispositif d'injection.

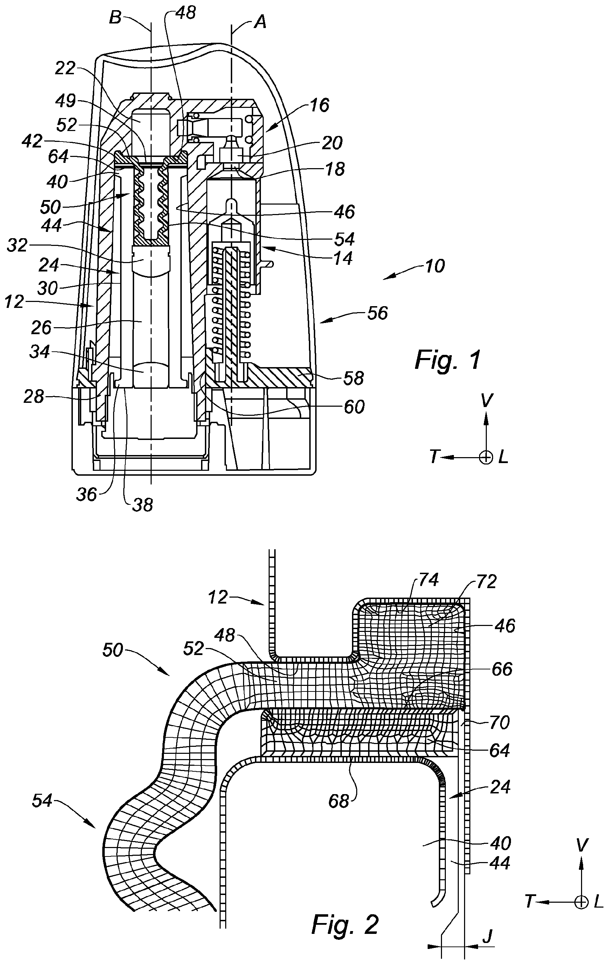

On a représenté à la figure 1 un dispositif 10 d'injection sans aiguille, ou seringue sans aiguille, qui comporte un corps 12 en forme de U comprenant successivement un dispositif de percussion 14, un générateur de gaz 16 comprenant une amorce 18 et une charge pyrotechnique 20, une chambre d'expansion 22, un réservoir 24 contenant le principe actif 26 liquide et une buse 28 d'injection.

Le dispositif de percussion 14 et le générateur de gaz 16 constituent un premier sous-ensemble linéaire du corps 12 qui s'étend axialement suivant un axe A de coulissement vertical, et le réservoir 24 contenant le principe actif 26 et la buse 28 d'injection forment un deuxième sous-ensemble linéaire du corps 12 qui s'étend axialement suivant un second axe B d'injection vertical.

Ces deux sous-ensembles sont reliés l'un à l'autre par la chambre d'expansion 22 qui a un axe perpendiculaire aux axes A, B des sous-ensembles.

Le réservoir 24 est constitué par un tube 30 en verre obturé par un piston amont 32 et un piston aval 34 entre lesquels est contenu le principe actif 26 liquide, les pistons étant réalisés en matériau déformable élastiquement à base d'élastomère.

Le réservoir 24 s'étend axialement depuis une collerette inférieure 36 qui présente une face inférieure 38 annulaire agencée en regard de la buse 28 d'injection, jusqu'à une collerette supérieure 40 présentant une face supérieure 42 annulaire.

Le réservoir 24 est agencé dans un logement 44 formé par le corps 12, logement 44 qui est délimité radialement par une paroi 46 tubulaire qui s'étend autour de l'axe B d'injection.

Le logement 44 s'étend axialement depuis un siège 48 radial supérieur qui est formé par le corps 12 et qui délimite un orifice de sortie 49 de la chambre d'expansion 22.

Selon un exemple de réalisation préféré, le corps 12 est réalisé par moulage en injection de matière plastique.

Aussi, selon la figure 1, le dispositif 10 est équipé d'une membrane 50 déformable élastiquement de forme globalement en T, qui comprend un disque 52 annulaire radial qui est interposé axialement entre la collerette supérieure 40 du réservoir 24 et le siège 48 formé par le corps 12, et une partie tubulaire 54 qui s'étend axialement dans le réservoir 24, depuis le disque 52 annulaire.

La partie tubulaire 54 de la membrane 50 est conçue pour s'étendre axialement, sous l'effet de la pression du gaz généré par le générateur de gaz 16, pour pousser le piston amont 32.

En référence à la figure 1, le corps 12 est enveloppé par un capot 56 creux qui délimite une ouverture inférieure fermée par une semelle 58 horizontale formant fond de capot.

La semelle 58 délimite un passage circulaire 60 autour de l'axe B d'injection qui est adapté pour le passage de la buse 28 d'injection et de l'extrémité aval du corps 12, de sorte que la buse 28 comporte un tronçon inférieur faisant saillie verticalement vers le bas hors du capot 56.

Plus particulièrement, la buse 28 est vissée sur une extrémité libre débouchante du logement 44 formé par le corps 12, la buse 28 comprimant axialement l'ensemble formé par le réservoir 24 et la membrane 50 sur le siège 48 du logement 44.

Aussi, le dispositif 10 d'injection est équipé d'un bouchon 62 qui est monté sur le corps 12 de façon amovible par un moyen de verrouillage du type à baïonnette.

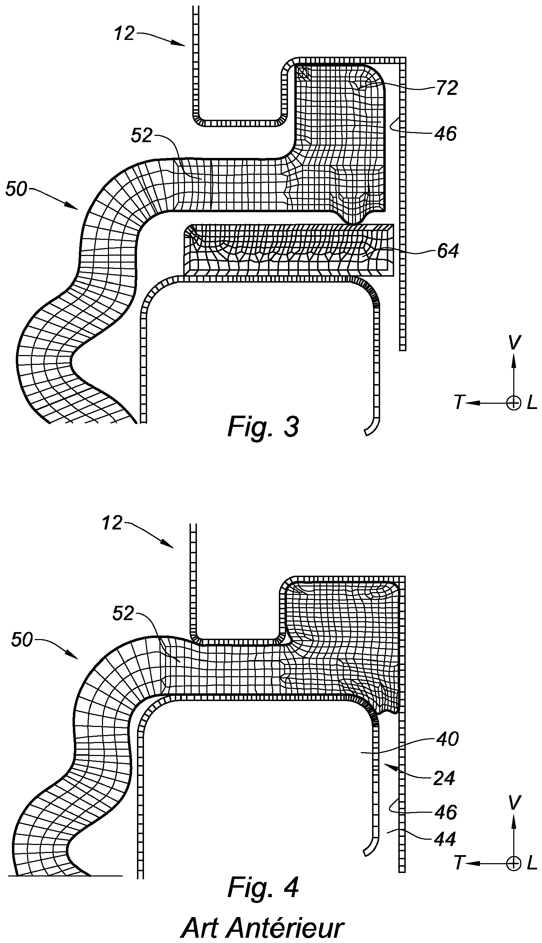

Conformément à l'invention, le dispositif 10 comporte une rondelle 64 annulaire anti-extrusion, illustrée aux figures 2 et 3, qui s'étend autour de la partie tubulaire 54 de la membrane 50 et qui est interposée axialement entre le disque 52 annulaire de la membrane 50 et la collerette supérieure 40 du réservoir 24.

La rondelle 64 anti-extrusion est délimitée axialement par une face supérieure 66 en appui sur le disque 52 de la membrane 50 et une face inférieure 68 en appui sur la collerette supérieure 40 du réservoir 24.

La rondelle 64 s'étend radialement globalement jusqu'à la paroi 46 tubulaire du logement 44 du corps 12, pour limiter le fluage axiale du disque 52 de la membrane 50 entre la collerette supérieure 40 du réservoir 24 et la paroi 46 tubulaire du logement 44, par déformation, lorsque le disque 52 de la membrane 50 est comprimé axialement.

A cet effet, la rondelle 64 annulaire est délimitée radialement par un bord périphérique 70 qui présente un jeu J radial, illustré à la figure 2, avec la paroi 46 tubulaire du logement 44 associé.

De préférence, le jeu J radial est compris entre 0,075 et 0,025 millimètre, suivant le rayon de la rondelle 64.

Aussi, selon un exemple de réalisation préféré, la rondelle 64 présente une épaisseur axiale de 0,6 millimètre.

Comme on peut le voir à la figure 2, le disque 52 de la membrane 50 forme un cordon 72 annulaire qui fait saillie axialement vers le haut et qui est logé dans une gorge 74 annulaire qui est délimitée par le siège 48 du corps 12.

La gorge 74 annulaire présente une forme complémentaire à celle du disque 52 de la membrane 50.

En effet, le disque 52 de la membrane 50 est adapté pour être agencé sans jeu, ou serré, dans l'espace délimité entre le siège 48 formé par le corps 12, la collerette supérieure 40 du réservoir 24 et la paroi 46 tubulaire du logement 44 formé par le corps 12, comme on peut le voir à la figure 2, lorsque la buse 28 d'injection est vissées sur le corps 12 suivant un couple nominal.

Le couple de serrage nominal de la buse 28 est de préférence d'au moins 120 Newtons.

A titre indicatif, on a représenté à la figure 3 la membrane 50 dans un état de repos avant le serrage de la buse 28, étant dans lequel la membrane 50 n'est pas comprimée, le disque 52 de la membrane 50 étant alors libre et agencé a jeu radial avec la paroi 46 du logement 44 et à jeu axial avec le siège 48 du corps 12.

Comme on peut le voir à la figure 2, le jeu radial réduit entre la rondelle 64 anti-extrusion et la paroi 46 du logement 44 empêche l'expansion du disque 52 de la membrane 50.

A l'inverse, en référence à la figure 4 qui illustre l'art antérieur, sans rondelle 64 anti-extrusion, le disque 52 de la membrane 50 tend à fluer et à s'épandre axialement dans le jeu délimité entre la paroi 46 et le réservoir 24.

En effet, un jeu fonctionnel radial est nécessaire entre le réservoir 24 et la paroi 46 du logement 44 pour favoriser le montage du réservoir 24 dans son logement 44.

Ce jeu fonctionnel est la conséquence des tolérances de réalisation du réservoir 24 en verre formé à chaud et du corps 12 injecté. Ce jeu fonctionnel peut varier de 0,15 à 0,55 millimètre, par exemple.

On constate que ce fluage de la membrane 50 limite la compression du disque 52 de la membrane 50, le disque 52 risquant de se déformer, notamment au cours de la décharge de gaz par le générateur 16 de gaz qui génère une pression d'injection globalement égale à 250 bars, et de créer des fuites entre la membrane 50 et le corps 12.

## Revendications

1. Dispositif (10) d'injection sans aiguille comportant :
- un corps (12) formant un logement (44) qui est délimité par une paroi (46) tubulaire et qui s'étend axialement depuis un siège (48) radial supérieur,
- un générateur de gaz (16),
- un réservoir (24) tubulaire qui renferme un principe actif (26) et qui s'étend axialement dans ledit logement (44) depuis une extrémité supérieure formant une collerette supérieure (40), jusqu'à une extrémité inférieure (36),
- une membrane (50) déformable élastiquement de forme globalement en T, qui comprend un disque (52) annulaire radial qui est interposé axialement entre la collerette supérieure (40) du réservoir (24) et ledit siège (48) formé par le corps (12), et une partie tubulaire (54) qui s'étend axialement dans le réservoir (24), depuis le disque (52) annulaire, et
- une buse (38) d'injection du principe actif (26) qui est agencée à l'extrémité inférieure du réservoir (24),
**caractérisé en ce qu'**il comporte une rondelle (64) annulaire qui est interposée axialement entre le disque (52) annulaire de la membrane (50) et la collerette supérieure (40) du réservoir (24), et qui s'étend radialement globalement jusqu'à la paroi (46) tubulaire du logement (24) du corps (12), pour limiter le fluage axiale du disque (52) de la membrane (50) entre le réservoir (24) et la paroi (46) tubulaire dudit logement (44), lorsque le disque (52) de la membrane (50) est comprimé axialement.

2. Dispositif (10) d'injection sans aiguille selon la revendication 1, **caractérisé en ce que** la rondelle (64) annulaire est délimitée radialement par un bord périphérique (70) qui présente un jeu (J) radial avec la paroi (46) tubulaire du logement (44) associé, le jeu radial étant compris entre 0,075 et 0,025 millimètre suivant le rayon de la rondelle (64).

3. Dispositif (10) d'injection sans aiguille selon l'une quelconque des revendications précédentes, **caractérisé en ce que** le disque (52) de la membrane (50) forme un cordon (72) annulaire qui fait saillie axialement vers le haut et qui est logé dans une gorge (74) de forme complémentaire qui est délimitée par le siège (48) du corps (12).

4. Dispositif (10) d'injection sans aiguille selon l'une quelconque des revendications précédentes, **caractérisé en ce que** le disque (52) de la membrane (50) est adapté pour être agencé sans jeu dans l'espace délimité entre le siège (48) formé par le corps (12), la collerette supérieure (40) du réservoir (24) et la paroi (46) tubulaire du logement (44) formé par le corps (12).

5. Dispositif (10) d'injection sans aiguille selon l'une quelconque des revendications précédentes, **caractérisé en ce que** le corps (12) est réalisé par moulage en injection.

6. Dispositif (10) d'injection sans aiguille selon l'une quelconque des revendications précédentes, **caractérisé en ce que** la membrane (50) est réalisée en matériau déformable élastiquement à base d'élastomère.

7. Dispositif (10) d'injection sans aiguille selon l'une quelconque des revendications précédentes, **caractérisé en ce que** la buse (28) d'injection est vissée sur une extrémité libre débouchante du logement (44) formé par le corps (12), la buse (28) comprimant axialement l'ensemble formé par le réservoir (24), la membrane (50) et la rondelle (64) sur le siège (48) du logement (44).

8. Dispositif (10) d'injection sans aiguille selon la revendication 7, **caractérisé en ce que** la buse (28) est vissée suivant un couple de serrage d'au moins 120 Newtons.

9. Dispositif (10) d'injection sans aiguille selon l'une quelconque des revendications précédentes, **caractérisé en ce que** le principe actif (26) contenu dans le réservoir (24) est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

## Patentansprüche

1. Nadellose Einspritzvorrichtung (10), die Folgendes umfasst:
- einen Körper (12), der eine Aufnahme (44) bildet, die durch eine röhrenförmige Wand (46) abgegrenzt ist und sich axial von einem radialen oberen Sitz (48) erstreckt,
- einen Gasgenerator (16),
- einen röhrenförmigen Behälter (24), der einen Wirkstoff (26) einschließt, und der sich axial in der Aufnahme (44) von einem oberen Ende, das einen oberen Bund (40) bildet, bis zu einem unteren Ende (36) erstreckt,
- eine elastisch verformbare Membran (50) insgesamt in T-Form, die eine radiale ringförmige Scheibe (52) umfasst, die axial zwischen dem oberen Bund (40) des Behälters (24) und dem Sitz (48), der von dem Körper (12) gebildet wird, eingefügt ist, und einen röhrenförmigen Teil (54), der sich axial in den Behälter (24) ausgehend von der ringförmigen Scheibe (52) erstreckt, und
- eine Einspritzdüse (38) des Wirkstoffs (26), die an dem unteren Ende des Behälters (24) eingerichtet ist,
**dadurch gekennzeichnet, dass** sie eine ringförmige Unterlegscheibe (64) umfasst, die axial zwischen der ringförmigen Scheibe (52) der Membran (50) und dem oberen Bund (40) des Behälters (24) eingefügt ist und sich radial insgesamt bis zu der röhrenförmigen Wand (46) der Aufnahme (24) des Körpers (12) erstreckt, um das axiale Kriechen der Scheibe (52) der Membran (50) zwischen dem Behälter (24) und der röhrenförmigen Wand (46) der Aufnahme (44) einzuschränken, wenn die Scheibe (52) der Membran (50) axial komprimiert wird.

2. Nadellose Einspritzvorrichtung (10) nach Anspruch 1, **dadurch gekennzeichnet, dass** die ringförmige Unterlegscheibe (64) radial durch einen umfänglichen Rand (70) abgegrenzt ist, der ein radiales Spiel (J) mit der röhrenförmigen Wand (46) der dazugehörenden Aufnahme (44) aufweist, wobei das radiale Spiel zwischen 0,075 und 0,025 Millimeter entlang des Radius der Unterlegscheibe (64) liegt.

3. Nadellose Einspritzvorrichtung (10) nach einem der vorstehenden Ansprüche, **dadurch gekennzeichnet, dass** die Scheibe (52) der Membran (50) einen ringförmigen Wulst (72) bildet, der axial nach oben vorragt und in einer Nut (74) mit komplementärer Form, die von dem Sitz (48) des Körpers (12) abgegrenzt ist, aufgenommen ist.

4. Nadellose Einspritzvorrichtung (10) nach einem der vorstehenden Ansprüche, **dadurch gekennzeichnet, dass** die Scheibe (52) der Membran (50) angepasst ist, um ohne Spiel in dem Raum eingerichtet zu sein, der zwischen dem Sitz (48), der von dem Körper (12) gebildet wird, dem oberen Bund (40) des Behälters (24) und der röhrenförmigen Wand (46) der Aufnahme (44), die von dem Körper (12) gebildet wird, abgegrenzt ist.

5. Nadellose Einspritzvorrichtung (10) nach einem der vorstehenden Ansprüche, **dadurch gekennzeichnet, dass** der Körper (12) durch Spritzguss geformt ist.

6. Nadellose Einspritzvorrichtung (10) nach einem der vorstehenden Ansprüche, **dadurch gekennzeichnet, dass** die Membran (50) aus einem elastisch verformbaren Material auf Elastomerbasis hergestellt ist.

7. Nadellose Einspritzvorrichtung (10) nach einem der vorstehenden Ansprüche, **dadurch gekennzeichnet, dass** die Einspritzdüse (28) auf ein freies nach außen mündendes Ende der Aufnahme (44) geschraubt ist, die von dem Körper (12) gebildet wird, wobei die Düse (28) die Einheit, die von dem Behälter (24), der Membran (50) und der Unterlegscheibe (64) gebildet wird, auf dem Sitz (48) der Aufnahme (44) axial komprimiert.

8. Nadellose Einspritzvorrichtung (10) nach Anspruch 7, **dadurch gekennzeichnet, dass** die Düse (28) gemäß einem Anziehdrehmoment von mindestens 120 Newton festgeschraubt ist.

9. Nadellose Einspritzvorrichtung (10) nach einem der vorstehenden Ansprüche, **dadurch gekennzeichnet, dass** der Wirkstoff (26), der in dem Behälter (24) enthalten ist, aus der Gruppe ausgewählt ist, die folgende Wirkstoffe umfasst:
- Methotrexat,
- Adrenalin,
- Sumatriptan,
- Hydrocortison,
- Naloxon,
- Midazolam,
- Apomorphin,
- Ethylnaltrexon-Bromid,
- Phytomenadion,
- Chlorpromazin-Hydrochlorid,
- Zuclopenthixol-Acetat,
- Danaparoid-Natrium,
- Enoxaparin-Natrium,
- Estradiol-Cypionat,
- Medroxyprogesteron-Acetat,
- Medroparin-Calcium,
- Methylprednisolon-Acetat,
- Heparin-Calcium,
- Terbutalin.

## Claims

1. A needleless injection device (10) including:
- a body (12) forming a housing (44) which is delimited by a tubular wall (46) and which extends axially from an upper radial seat (48),
- a gas generator (16),
- a tubular reservoir (24) which contains an active ingredient (26) and which extends axially into said housing (44) from an upper end forming an upper collar (40), to a lower end (36),
- an elastically-deformable diaphragm (50) with a T-like general shape, which comprises a radial annular disk (52) which is axially interposed between the upper collar (40) of the reservoir (24) and said seat (48) formed by the body (12), and a tubular portion (54) which extends axially into the reservoir (24), from the annular disk (52), and
- a nozzle (38) for injecting the active ingredient (26) which is arranged at the lower end of the reservoir (24),
**characterized in that** it includes an annular washer (64) which is axially interposed between the annular disk (52) of the diaphragm (50) and the upper collar (40) of the reservoir (24), and which extends radially generally up to the tubular wall (46) of the housing (24) of the body (12), so as to limit the axial creeping of the disk (52) of the diaphragm (50) between the reservoir (24) and the tubular wall (46) of said housing (44), when the disk (52) of the diaphragm (50) is axially compressed.

2. The needleless injection device (10) according to claim 1, **characterized in that** the annular washer (64) is radially delimited by a peripheral edge (70) which has a radial clearance (J) with the tubular wall (46) of the associated housing (44), the radial clearance being comprised between 0.075 and 0.025 millimeter depending on the radius of the washer (64).

3. The needleless injection device (10) according to any one of the preceding claims, **characterized in that** the disk (52) of the diaphragm (50) forms an annular bead (72) which projects axially upwards and which is housed within a groove (74) with a matching shape which is delimited by the seat (48) of the body (12).

4. The needleless injection device (10) according to any one of the preceding claims, **characterized in that** the disk (52) of the diaphragm (50) is adapted to be arranged without any clearance in the space delimited between the seat (48) formed by the body (12), the upper collar (40) of the reservoir (24) and the tubular wall (46) of the housing (44) formed by the body (12).

5. The needleless injection device (10) according to any one of the preceding claims, **characterized in that** the body (12) is made by injection molding.

6. The needleless injection device (10) according to any one of the preceding claims, **characterized in that** the diaphragm (50) is made of an elastomer-based elastically-deformable material.

7. The needleless injection device (10) according to any one of the preceding claims, **characterized in that** the injection nozzle (28) is screwed on a through free end of the housing (44) formed by the body (12), the nozzle (28) axially compressing the set formed by the reservoir (24), the diaphragm (50) and the washer (64) on the seat (48) of the housing (44).

8. The needleless injection device (10) according to claim 7, **characterized in that** the nozzle (28) is screwed at a tightening torque of at least 120 Newtons.

9. The needleless injection device (10) according to any one of the preceding claims, **characterized in that** the active ingredient (26) contained in the reservoir (24) is selected from the group comprising the following active ingredients:
- Methotrexate,
- Adrenaline,
- Sumatriptan,
- Hydrocortisone,
- Naloxone,
- Midazolam,
- Apomorphine,
- Ethylnaltrexone bromide,
- Phytomenadione,
- Chlorpromazine hydrochloride,
- Zuclopenthixol acetate,
- Danaparoid sodium,
- Enoxaparin sodium,
- Estradiol cypionate,
- Medroxyprogesterone acetate,
- Medroparin calcium,
- Methylprednisolone acetate,
- Heparin calcium,
- Terbuline.
